# EUROPEAN PATENT APPLICATION

(11) **EP 3 933 276 A1**
(43) Date of publication of application: **05.01.2022**
(21) Application number: 21176421.2
(22) Date of filing: 28.05.2021
(51) Int. Cl.: F24C 14/00, F24C 15/08, H05B 6/64, A61L 2/10, A23L 3/28, A61L 9/20

(54) **A HOUSEHOLD APPLIANCE COMPRISING A UV LAMP**

(30) Priority: 29.06.2020 TR 202010238
(71) Applicant: Arçelik Anonim Sirketi, 34445 Istanbul (TR)
(72) Inventor: CHAUDRY, Danyal Amin, 34445 ISTANBUL (TR); OZDOGAN, Melih, 34445 ISTANBUL (TR); USTA, Kadir, 34445 ISTANBUL (TR); EKER, Tevfik, 34445 ISTANBUL (TR); OFLUGIL, ILHAN, 34445 ISTANBUL (TR)

(57) **Abstract**

The present invention relates to a household appliance (1) comprising a body (2); a top wall (3) which is provided at the upper side of the body (2); a housing (4) which is provided on the top wall (3); a connection member (5) which is positioned on the top wall (3) and which is sized so as to fit into the housing (4); and a UV lamp module (6) which is disposed on the connection member (5), wherein the household appliance further comprises

- at least one first recess (7) which is provided on the connection member (5) and at least one first protrusion (8) which is adjacent to the periphery of the housing (4) and which engages with the first recess (7) so as to ensure the fixing of the connection member (5), and

- at least one second protrusion (9) which is provided on the connection member (5) and at least one second recess (10) which is provided on the UV lamp module (6) and which engages with the second protrusion (9).

## Description

The present invention relates to a household appliance with improved safety and ease of assembly.

Recently, it has become important to design the most useful and safe configuration for the user in the assembly of household appliances. Today, it is aimed to correctly position ultraviolet light sources such as lamps used in household appliances for various purposes in ovens, microwave ovens, and disinfection devices so as to maximize the safety of the user. Especially in case the users get in contact with different parts of said devices while cleaning or touching, factors harmful to health such as ultraviolet rays, which create a high risk in case of human exposure occur. It is highly required to position the ultraviolet lamps so as to be firmly fixed to the household appliance in a manner not to be easily accessed by the users. It is clear that a solution is required for an assembly group having a simple connection structure which can provide convenience without additional labor and labor cost in the assembly of the device body.

In the state of the art European Patent Application No. EP1464895A1, an assembly arrangement is disclosed, which is composed of two pieces produced from sheet metal so as to be used in cooking devices and which is enabled to be bent and held by means of an intermediate piece.

The state of the art Chinese Patent Application No. CN201475625 discloses the formation of a convex platform by means of a flange structure which increases the rigidity of the assembly, which is adjacent to the rear wall on air conditioners.

The aim of the present invention is the realization of a household appliance wherein the labor loss is partially prevented at the junction of the body and the rear wall and the safety of use is increased.

The household appliance realized in order to attain the aim of the present invention, explicated in the claims, comprises a body; a top wall which is provided at the upper side of the body; a housing which is provided on the top wall; a connection member which is positioned on the top wall and which is sized so as to fit into the housing; and a UV lamp module which is disposed on the connection member. The connection member which is an intermediate component is placed onto the housing formed as a cut-out on the top wall for the placement of the UV lamp.

The household appliance comprises at least one first recess which is provided on the connection member and at least one protrusion which is adjacent to the periphery of the housing and which engages with the first recess so as to ensure the fixing of the connection member. By pushing the connection member towards the top wall in the vertical axis, the first recess and the first protrusion engage with each other and get snap fitted. The first recess provided around the housing provides a gapless and rigid gripping between the connection member and the top wall. Moreover, the household appliance comprises at least one second protrusion which is provided on the connection member and at least one second recess which is provided on the UV lamp module and which engages with the second protrusion. Thus, the UV lamp module, which is positioned such that the illuminating part thereof facing the body from the housing, can be tightly fixed onto the connection member. Consequently, the risk of the users contacting the ultraviolet rays is minimized. Moreover, by means of only two separate engaging structures, a significant ease of assembly and improvement of labor are provided without using any screw, welding, rivet, etc.

In an embodiment of the present invention, the household appliance comprises at least two first recesses which are positioned symmetrically around the housing. With respect to the horizontal and vertical axes passing through the center of the housing, the first recesses are symmetrically positioned. Thus, a better engagement is provided and rigidity is improved.

In an embodiment of the present invention, the household appliance comprises at least three different first recesses and at least three different first protrusions which engage with the first recesses. By means of the oppositely positioned three or more first recesses and protrusions, the support forces exerted by the connection member onto the upper body are increased.

In an embodiment of the present invention, the household appliance comprises at least three different second recesses and at least three different second protrusions which engage with the second recesses. By means of the oppositely positioned three or more second recesses and protrusions, the support forces at the regions where the connection member grips the UV lamp module are increased, and a structure which is more resistant to external factors is obtained.

In an embodiment of the present invention, the household appliance comprises the first protrusion having a t-shaped notch which squeezes the first recess and fixes the connection member after the connection member is placed. Thus, the first recesses snap fitting with the notch at the upper side create a more fixed and gapless structure.

In an embodiment of the present invention, the household appliance is a cooking device. The assembly of the UV lamp module which is used in devices such as oven, microwave oven, etc. and which provides disinfection becomes more efficient.

In an embodiment of the present invention, the household appliance is a disinfection device. The assembly of the UV lamp module which is used in disinfection devices and which provides disinfection becomes more efficient.

By means of the household appliance of the present invention, a cost-efficient solution which is safer for the user in the assembly of the UV lamp is realized.

A household appliance realized in order to attain the aim of the present invention is illustrated in the attached figures, where:

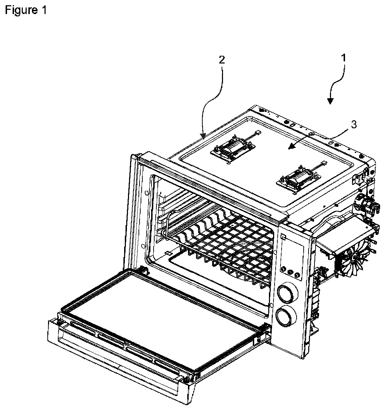

Figure 1 - is the perspective view of the household appliance.

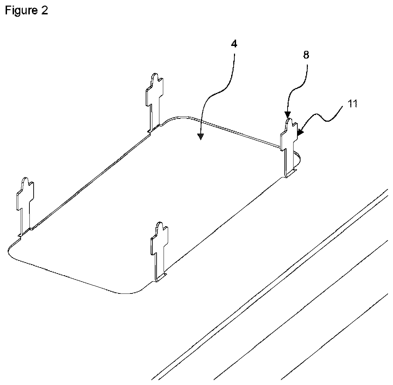

Figure 2 - is the detailed view of the housing.

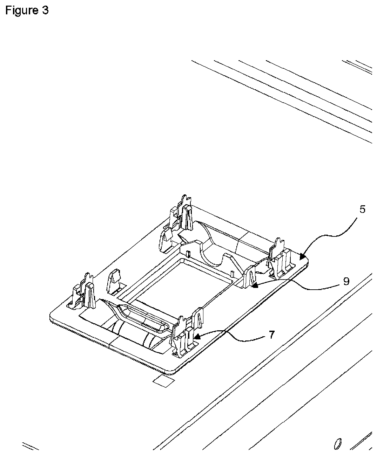

Figure 3 - is the detailed view of the connection member.

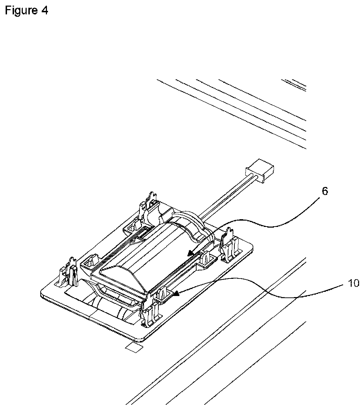

Figure 4 - is the detailed view of the UV lamp module.

The elements illustrated in the figures are numbered as follows:
1. Household appliance
2. Body
3. Top wall
4. Housing
5. Connection member
6. UV lamp module
7. First recess
8. First protrusion
9. Second protrusion
10. Second recess
11. Notch

The household appliance (1) comprises a body (2); a top wall (3) which is provided at the upper side of the body (2); a housing (4) which is provided on the top wall (3); a connection member (5) which is positioned on the top wall (3) and which is sized so as to fit into the housing (4); and a UV lamp module (6) which is disposed on the connection member (5). The body (2) is considered as the casing or the skeleton of the household appliance (1). The body (2) as well as the top wall (3) are generally produced from sheet metal. The connection member (5) and the UV lamp module (6) are generally produced from plastic material. The connection member (5) which is an intermediate component is placed onto the housing (4) which is formed as a cut-out on the top wall (3) for the placement of the UV lamp module (6) and which can be circular or polygonal. The household appliance (1) comprises at least one first recess (7) which is provided on the connection member (5) and at least one first protrusion (8) which is adjacent to the periphery of the housing (4) and which engages with the first recess (7) so as to ensure the fixing of the connection member (5). By pushing the connection member (5) towards the top wall (3) in the vertical axis, the first recess (7) and the first protrusion (8) engage with each other and get snap fitted. The first recess (7) provided around the housing (4) provides a gapless and rigid gripping between the connection member (5) and the top wall (3). Since generally the first recess (7) is plastic and the first protrusion (8) is metal, a more efficient solution is provided, which ensures better gripping in terms of material strength. Moreover, the household appliance (1) comprises at least one second protrusion (9) which is provided on the connection member (5) and at least one second recess (10) which is provided on the UV lamp module (6) and which engages with the second protrusion (9). Thus, the UV lamp module (6), which is positioned such that the illuminating part thereof facing the household appliance (1) from the housing (4), can be tightly fixed onto the connection member (5). Consequently, the UV lamp module (6), the connection member (5) and the top wall (3) are rigidly fixed to each other at the outside of the body (2) so as not to be accessed by the user. By means of only two separate engaging structures, a significant ease of assembly and improvement of labor are provided without using any screw, welding, rivet, etc. When the household appliance (1) is required to be repaired, the service staff can easily access the ultraviolet lamp components on the casing by removing the outer cover.

In an embodiment of the present invention, the household appliance (1) comprises at least two first recesses (7) which are positioned symmetrically around the housing (4). With respect to the horizontal and vertical axes passing through the center of the housing (4), the first recesses (7) are symmetrically positioned. For example, for a quadrilateral housing (4), a four-piece support structure is formed by means of the first recesses (7) symmetrically positioned on the opposite sides. This number can be increased for different housing (4) geometries. Thus, a better engagement is provided and rigidity is improved.

In an embodiment of the present invention, the household appliance comprises (1) at least three different first recesses (7) and at least three different first protrusions (8) which engage with the first recesses (7). For example, a rectangular housing (4) comprises four first recesses (7) and four first protrusions (8), one on each of the long sides and short sides. By means of the oppositely positioned three or more first recesses (7) and protrusions (8), the support forces exerted by the connection member (5) onto the top wall (3) are increased, and a structure which is more resistant to external factors is obtained.

In an embodiment of the present invention, the household appliance (1) comprises at least three different second recesses (10) and at least three different second protrusions (9) which engage with the second recesses (10). A design suitable for the geometry of the connection member (5) can be realized at three or more points. By means of the oppositely positioned three or more second recesses (10) and protrusions (9), the support forces at the regions where the connection member (5) grips the UV lamp module (6) are increased, and a structure which is more resistant to external factors is obtained.

In an embodiment of the present invention, the household appliance (1) comprises the first protrusion (8) having a t-shaped notch (11) which squeezes the first recess (7) and fixes the connection member (5) after the connection member (5) is placed. The notch (11) is basically in the form of a "t" or "+" and represents a structure which is provided between the two ends of the first protrusion (8) and which is thicker than the remaining part. By means of the first recess (7) enclosing the first protrusion (8), the notch (11) fits into the upper side of the first recess (7). Thus, the first recesses (7) snap fitting with the notch (11) at the upper side create a more fixed and gapless structure.

In an embodiment of the present invention, the household appliance (1) is a cooking device. The assembly of the UV lamp module (6) which is used in devices such as oven, microwave oven, etc. and which provides disinfection becomes more efficient.

In an embodiment of the present invention, the household appliance (1) is a disinfection device. The assembly of the UV lamp module (6) which is used in disinfection devices and which provides disinfection becomes more efficient.

In the household appliance (1) of the present invention, by means of the connection features in the mounting of the UV lamp, a more efficient assembly process is provided for the producer while ensuring better safety of use for the user.

## Claims

1. A household appliance (1) comprising a body (2); a top wall (3) which is provided at the upper side of the body (2); a housing (4) which is provided on the top wall (3); a connection member (5) which is positioned on the top wall (3) and which is sized so as to fit into the housing (4); and a UV lamp module (6) which is disposed on the connection member (5), **characterized by**
- at least one first recess (7) which is provided on the connection member (5) and at least one first protrusion (8) which is adjacent to the periphery of the housing (4) and which engages with the first recess (7) so as to ensure the fixing of the connection member (5), and
- at least one second protrusion (9) which is provided on the connection member (5) and at least one second recess (10) which is provided on the UV lamp module (6) and which engages with the second protrusion (9).

2. A household appliance (1) as in Claim 1, **characterized by** at least two first recesses (7) which are positioned symmetrically around the housing (4).

3. A household appliance (1) as in Claim 1 or 2, **characterized by** at least three different first recesses (7) and at least three different first protrusions (8) which engage with the first recesses (7).

4. A household appliance (1) as in any one of the above claims, **characterized by** at least three different second recesses (10) and at least three different second protrusions (9) which engage with the second recesses (10).

5. A household appliance (1) as in any one of the above claims, **characterized by** the first protrusion (8) having a t-shaped notch (11) which squeezes the first recess (7) and fixes the connection member (5) after the connection member (5) is placed.

6. A household appliance (1) as in any one of the above claims, which is a cooking device.

7. A household appliance (1) as in any one of the above claims, which is a disinfection device.
